Europäisches Patentamt

European Patent Office

Office européen des brevets

(11) Veröffentlichungsnummer: **0 337 127 B1**

(12) **EUROPÄISCHE PATENTSCHRIFT**

(45) Veröffentlichungstag der Patentschrift: **21.04.93**

(51) Int. Cl.5: **C07C 21/18**, C07C 17/24

(21) Anmeldenummer: **89104350.7**

(22) Anmeldetag: **11.03.89**

(54) **Verfahren zur Herstellung von Hexafluorpropen.**

(30) Priorität: **14.03.88 DE 3808437**
**09.07.88 DE 3823370**

(43) Veröffentlichungstag der Anmeldung:
**18.10.89 Patentblatt 89/42**

(45) Bekanntmachung des Hinweises auf die
Patenterteilung:
**21.04.93 Patentblatt 93/16**

(84) Benannte Vertragsstaaten:
**BE DE FR GB IT NL**

(56) Entgegenhaltungen:
**DE-B- 1 229 515**
**DE-B- 1 236 497**
**US-A- 3 446 858**

(73) Patentinhaber: **HOECHST AKTIENGESELL-**
**SCHAFT**
**Postfach 80 03 20**
**W-6230 Frankfurt am Main 80(DE)**

(72) Erfinder: **Freudenreich, Reinhold**
**Ortlehnerstrasse 1**
**W-8269 Burgkirchen(DE)**
Erfinder: **Mielke, Ingolf, Dr.**
**Hochstaufenstrasse 16**
**W-8269 Burgkirchen(DE)**
Erfinder: **Rettenbeck, Karl**
**Kasten 7**
**W-8269 Burgkirchen(DE)**
Erfinder: **Schöttle, Thomas, Dr.**
**Talhauserstrasse 25**
**W-8269 Burgkirchen(DE)**

EP 0 337 127 B1

**Beschreibung**

Die Erfindung betrifft ein Verfahren zur Herstellung von Hexafluorpropen nach Anspruch 1.

Hexafluorpropen findet zunehmende Anwendung als Comonomeres für die technische Herstellung von Polymerisaten auf Basis von Tetrafluorethen. Es besteht daher die Aufgabe, kostengünstige Herstellmethoden für Hexafluorpropen zu entwickeln. Es ist bekannt, Hexafluorpropen durch thermische Behandlung von weitgehend fluorierten Kohlenwasserstoffen bei Temperaturen von 600 bis 1 200 °C herzustellen.

Miller beschreibt in "Preparation, Properties and Technology of Fluorine and Organic Fluoro Compounds", McGraw-Hill, New York, 1951, Seiten 592 und 593 die thermische Behandlung von Tetrafluorethen. Bei 655 °C wird unter Normaldruck Hexafluorpropen mit einer Ausbeute von 42 % erhalten, während bei 750 °C die Ausbeute an Hexafluorpropen stark abnimmt und in überwiegendem Maße Octafluorbuten-(1) entsteht.

Aus US 2,758,138 ist die Herstellung von Hexafluorpropen durch thermische Behandlung von Tetrafluorethen bei 750 bis 900 °C und einem Druck von 25 bis 200 mm Hg (3,3 bis 26,7 kPa) bekannt, wobei je $dm^3$ Inhalt der Reaktionszone 20 bis 5 000 g/h Tetrafluorethen eingespeist werden. Anstelle des reinen Tetrafluorethens kann auch eine Mischung aus Tetrafluorethen und Hexafluorethan verwendet werden, wobei letzteres mit nicht-umgesetztem und neu zugesetztem Tetrafluorethen wieder in die Reaktionszone zurückgeführt wird.

US 2,970,176 beschreibt ein ähnliches Verfahren, bei dem im Druckbereich von 0,2 bis 65 psi (1,38 bis 448,2 kPa) gearbeitet wird. Anstelle des reinen Tetrafluorethens wird eine Mischung verwendet, die auf 1 mol Tetrafluorethen mindestens 0,05 mol höher siedender fluorierter Kohlenwasserstoffe der Formeln

$$C_nF_{2n+2}; \ C_{n+3}F_{2(n+3)} \ und \ C_{n+3}F_{2(n+2)} \ ,$$

worin n = 1 bis 10 ist, enthält. Die genannten fluorierten Kohlenwasserstoffe können Nebenprodukte der Pyrolyse von Tetrafluorethen sein. Sie werden unter Ergänzung des verbrauchten Tetrafluorethens wieder in die thermische Behandlung zurückgeführt.

Nach US 3,446,858 wird Hexafluorpropen durch Pyrolyse von Tetrafluorethen und/oder Octafluorcyclobutan in Gegenwart von 50 bis 95 Mol-% Wasserdampf unter adiabatischen Bedingungen bei 700 bis 900 °C hergestellt, wobei die Pyrolyse-Temperatur durch vorherige Mischung der Reaktionsteilnehmer mit überhitztem Wasserdampf eingestellt wird. Dieses Verfahren liefert gute Ausbeuten an Hexafluorpropen, erfordert aber die apparativ und energetisch aufwendige, destillative Trennung des bei der Pyrolyse von Chlordifluormethan zur Gewinnung von Tetrafluorethen anfallenden Gemisches von Chlortetrafluorethan und Octafluorcyclobutan sowie auch die apparativ aufwendige Zumischung hocherhitzten Wasserdampfes, die bei dem weiter unten beschriebenen neuen Verfahren zwar möglich aber nicht notwendig ist.

Nach DE 23 29 750-C2 wird Hexafluorpropen durch Pyrolyse eines Gemisches von Tetrafluorethen und Kohlendioxid bei 790 bis 850 °C und einem Druck von 0,75 bis 2 Atmosphären (73,6 bis 196,2 kPa) hergestellt, wobei der Partialdruck des Tetrafluorethens mindestens 360 mm Hg (48,0 kPa) betragen soll.

Krasnov, Il'ina und Polunina, Nauchn. Tr.-Permsk. Politekh. Inst., 185 (1976), Seiten 11 bis 14 (CA Vol. 91, Nr. 4830x) beschreiben die Pyrolyse von Chlortetrafluorethan bei 700 bis 850 °C, wobei als Hauptprodukte 1,1-Difluorethen und Hexafluorpropen entstehen.

Barabanov, Volkov, V'yunov und Maksimov, Zh. Obshch. Khim. 55 (4) (1985), Seiten 868 bis 871 (CA Vol. 103, Nr. 141257w) beschreiben die Pyrolyse von 1-Chlor-1,1,2,2,3,3-hexafluorpropan bei 740 bis 800 °C, wobei Tetrafluorethen, Hexafluorpropen und Chlortrifluorethen entstehen.

Aus DE-PS 1 229 515 ist die Herstellung von Hexafluorpropen durch thermische Spaltung von 2-Chlor-1,1,1,3,3,3-hexafluorpropan bei 650 bis 850 °C und Verweilzeiten von 3 bis 120 Sekunden bekannt. Die Ausbeute beträgt maximal 45 %.

Ferner ist aus DE-PS 1 236 497 die Herstellung von Hexafluorpropen durch gemeinsame Pyrolyse von Chlordifluormethan und 2-Chlor-1,1,1,2-tetrafluorethan im Verhältnis 1 : 1 bis 10 : 1 bei 500 bis 1 000 °C bekannt. Nach den Beispielen wird Hexafluorpropen mit Selektivitäten von 42,0 bis 79,0 % erhalten. Wenn man jedoch anstelle 2-Chlor-1,1,1,2-tetrafluorethan das bei der technischen Pyrolyse von Chlordifluormethan zu Tetrafluorethen als Nebenprodukt in überwiegendem Maße anfallende 1-Chlor-1,1,2,2-tetrafluorethan verwendet, treten Schwierigkeiten auf und es wird eine schlechte Selektivität der Hexafluorpropen-Bildung beobachtet, wie nachfolgender Vergleichsversuch zeigt.

Es wurde nun ein Verfahren gefunden, das es ermöglicht, Chlortetrafluorethan und/oder Chlorhexafluorpropan, insbesondere die bei der technischen Herstellung von Tetrafluorethen durch thermische Spaltung von Chlordifluormethan hauptsächlich als Nebenprodukt anfallenden Verbindungen 1-Chlor-1,1,2,2-tetrafluorethan und 1-Chlor-1,1,2,2,3,3-hexafluorpropan, oder Chlortetrafluorethan und Perfluorcyclobutan, insbeson-

2

dere das bei der technischen Herstellung von Tetrafluorethen durch thermische Spaltung von Chlordifluormethan als Nebenprodukt anfallende azeotrope Gemisch von Chlortetrafluorethan und Perfluorcyclobutan, bei guter Selektivität mit wesentlich verbesserter Raum-Zeit-Ausbeute zu Hexafluorpropen umzusetzen.

Das neue Verfahren zur Herstellung von Hexafluorpropen durch thermische Spaltung von Chlortetrafluorethan und/oder Chlorhexafluorpropan oder eines Gemisches von Chlortetrafluorethan und Perfluorcyclobutan bei einer Temperatur von 600 bis 1 000 ° C und einem Druck von 1 bis 1 000 kPa ist dadurch gekennzeichnet, daß die thermische Spaltung in Gegenwart von 0,05 bis 20 mol Tetrafluorethen auf 1 mol eingesetztes Chlortetrafluorethan und/oder Chlorhexafluorpropan oder Gemisch aus Chlortetrafluorethan und Perfluorcyclobutan erfolgt.

Für das neue Verfahren sind alle Isomeren des Chlortetrafluorethans beziehungsweise des Chlorhexafluorpropans geeignet. Gute Ergebnisse werden beispielsweise mit 2-Chlor-1,1,1,2-tetrafluorethan erhalten. Wegen ihrer leichten Beschaffbarkeit und guten Wirkung werden besonders bevorzugt die Verbindungen 1-Chlor-1,1,2,2-tetrafluorethan und 1-Chlor-1,1,2,2,3,3-hexafluorpropan verwendet. Es können auch Gemische eingesetzt werden, die entweder die beiden Isomeren des Chlortetrafluorethans oder Isomere des Chlorhexafluorpropans oder auch Isomere des Chlorhexafluorethans und Chlorhexafluorpropans enthalten. Bevorzugt sind solche Gemische, deren Gehalt an einer oder mehreren Verbindungen, in denen Wasserstoff und Chlor am selben Kohlenstoff gebunden sind, höchstens 20 Gew.-%, bezogen auf die eingesetzte Gesamtmenge Chlortetrafluorethan und/oder Chlorhexafluorpropan beträgt. Solche Gemische fallen beispielsweise bei der thermischen Zersetzung von Chlordifluormethan als Nebenprodukte an. Es ist üblich, diese Nebenprodukte durch fraktionierte Destillation vom Hauptprodukt Tetrafluorethen abzutrennen. Die thermische Spaltung solcher Destillate bei Temperaturen von 600 bis 1 000 ° C ergibt nur vergleichsweise schlechte Raum-Zeit-Ausbeuten in bezug auf die Bildung von Hexafluorpropen. Damit kann die Kapazität des Reaktors, in dem die thermische Spaltung von Chlortetrafluorethan und/oder Chlorhexafluorpropan stattfindet, nur ungenügend genutzt werden.

Für das neue Verfahren sind ferner Gemische aus Chlortetrafluorethen und Perfluorcyclobutan geeignet. Das molare Mischungsverhältnis von Chlortetrafluorethan und Perfluorcyclobutan beträgt vorteilhaft 1 : 10 bis 10 : 1 und insbesondere 1 : 3 bis 3 : 1. Wegen ihrer leichten Beschaffbarkeit und guten Wirkung werden besonders bevorzugt azeotrope Gemische von Chlortetrafluorethan und Perfluorcyclobutan verwendet.

Solche Gemische fallen beispielsweise bei der thermischen Zersetzung von Chlordifluormethan als Nebenprodukte an. Es ist üblich, diese Nebenprodukte durch fraktionierte Destillation vom Hauptprodukt Tetrafluorethen abzutrennen. Die thermische Spaltung der genannten azeotropen Gemische ohne Tetrafluorethen bei Temperaturen von 600 bis 1 000 ° C ergibt nur vergleichsweise schlechte Raum-Zeit-Ausbeuten in bezug auf die Bildung von Hexafluorpropen. Damit kann die Kapazität des Reaktors, in dem die thermische Spaltung des Gemisches aus Chlortetrafluorethan und Perfluorcyclobutan stattfindet, nur ungenügend genutzt werden.

Es können Gemische eingesetzt werden, die die beiden Isomere des Chlortetrafluorethans enthalten. Bevorzugt sind in diesem Fall solche Gemische, deren Gehalt an 2-Chlor-1,1,1,2-tetrafluorethan höchstens 20 Gew.-%, bezogen auf die eingesetzte Gesamtmenge, Chlortetrafluorethan beträgt.

Ab 0,05 mol Tetrafluorethen auf 1 mol eingesetztes Chlortetrafluorethan und/oder Chlorhexafluorpropan oder einem Gemisch aus Chlortetrafluorethan und Perfluorcyclobutan beginnt die erfindungsgemäße Wirkung des Tetrafluorethenzusatzes. Werden über 20 mol Tetrafluorethen auf 1 mol eingesetztes Chlortetrafluorethan und/oder Chlorhexafluorpropan oder einem Gemisch aus Chlortetrafluorethan und Perfluorcyclobutan verwendet, können Schwierigkeiten auftreten, beispielsweise dadurch, daß der Spaltreaktor thermisch instabil wird. Vorzugsweise werden 0,5 bis 5 mol und insbesondere 1 bis 3 mol Tetrafluorethen auf 1 mol eingesetztes Chlortetrafluorethan und/oder Chlorhexafluorpropan oder einem Gemisch aus Chlortetrafluorethan und Perfluorcyclobutan verwendet.

Die erfindungsgemäße thermische Spaltung findet bei Temperaturen von 600 bis 1 000 ° C, die an der Reaktorwand am Ende der Reaktionszone gemessen werden, statt. Unter 600 ° C wird im allgemeinen ein zu geringer Umsatz festgestellt. Über 1 000 ° C treten in zunehmendem Maße unerwünschte Nebenreaktionen auf, außerdem wird die thermische Spaltung bei so hohen Temperaturen unnötig kostspielig. Vorzugsweise wird bei Temperaturen von 700 bis 900 ° C und insbesondere bei 750 bis 860 ° C gearbeitet.

Der Druck des erfindungsgemäß einzusetzenden Gasgemisches am Reaktoreingang soll 1 bis 1 000 kPa betragen. Unterhalb 1 kPa werden im allgemeinen zu ungünstige Raum-Zeit-Ausbeuten erzielt, oberhalb 1 000 kPa sind im allgemeinen unnötig hohe Apparatekosten erforderlich, auch wird die Selektivität der Hexafluorpropenbildung ungünstiger und die Bildung von Nebenprodukten nimmt zu. Vorzugsweise wird bei einem Druck des erfindungsgemäß einzusetzenden Gemisches von 10 bis 200 kPa, insbesondere bei 20 bis 100 kPa, gearbeitet.

EP 0 337 127 B1

Die mittlere Verweilzeit des Gasgemisches im Bereich der thermischen Spaltung beträgt in Abhängigkeit von der gewählten Spalttemperatur zweckmäßig 0,01 bis 20 Sekunden. Bei höheren Spalttemperaturen, etwa im Bereich 850 bis 1 000 °C, wird man eine niedrigere Verweilzeit, beispielsweise im Bereich von 0,01 bis 1 Sekunde, bei niedrigen Spalttemperaturen, etwa im Bereich 600 bis 700 °C, wird man höhere Verweilzeiten, etwa im Bereich 0,1 bis 20 Sekunden, wählen. Gute Ergebnisse werden bei mittleren Verweilzeiten von 0,03 bis 7 erhalten. Sofern ein Gasgemisch, bestehend aus Tetrafluorethen, Chlortetrafluorethan und/oder Chlorhexafluorpropan, eingesetzt wird, sind mittlere Verweilzeiten von 0,08 bis 2 Sekunden, wenn ein Gasgemisch, bestehend aus Tetrafluorethen, Chlortetrafluorethan und Perfluorcyclobutan, eingesetzt wird, sind mittlere Verweilzeiten von 0,05 bis 1 Sekunden besonders bevorzugt. Bei mittleren Verweilzeiten über 20 Sekunden wird die Bildung unerwünschter Nebenprodukte begünstigt, auch tritt Belagsbildung an den Reaktoroberflächen, die mit dem thermisch zu spaltenden Gasgemisch in Berührung kommen, auf, die wiederum einen schlechteren Wärmeübergang zur Folge haben. Bei mittleren Verweilzeiten unter 0,01 Sekunde werden im allgemeinen zu geringe Umsätze beobachtet. Die vorgenannte mittlere Verweilzeit wird wie folgt ermittelt: Das Reaktorvolumen, in dem die thermische Spaltung stattfindet, wird durch das Gasvolumen, das die in einer Sekunde in den Reaktor eingetragene Gasmenge bei den im Reaktor herrschenden Temperatur- und Druckbedingungen einnimmt, dividiert.

Vorteilhaft können je 1 mol des erfindungsgemäß einzusetzenden Gasgemisches aus Tetrafluorethen, Chlortetrafluorethan und/oder Chlorhexafluorpropan oder des Gasgemisches aus Tetrafluorethen, Chlortetrafluorethan und Perfluorcyclobutan vor der thermischen Spaltung 0,01 bis 20 mol, insbesondere 0,1 bis 3 mol, von mindestens einem Inertgas zugesetzt werden. Das Inertgas kann vor dem Zusatz auf eine erhöhte Temperatur, beispielsweise 500 bis 1 000 °C erwärmt worden sein. Vorteilhaft wird als Inertgas ein nicht-fluorierter Stoff oder ein Gemisch mehrerer nicht-fluorierter Stoffe verwendet, der (das) unter den Bedingungen der thermischen Spaltung gasförmig ist und chemisch nicht reagiert. Beispiele für geeignete Stoffe sind Stickstoff, Argon, Kohlendioxid und insbesondere Wasser. Wenn dem erfindungsgemäßen Gasgemisch Inertgase zugesetzt werden, beziehen sich die weiter oben angeführten Druckangaben auf den Partialdruck des erfindungsgemäß einzusetzenden Gasgemisches.

Der Reaktor, in dem die thermische Spaltung stattfindet, kann verschiedene Gestalt haben. Er kann beispielsweise ein einfaches Rohr sein, das von außen erwärmt wird, oder ein Reaktor, wie er in der französischen Patentschrift Nr. 1 354 341 beschrieben ist, bei dem die thermisch zu spaltenden gasförmigen fluorierten Verbindungen im wesentlichen durch das erhitzte zugeführte Inertgas auf die Temperatur gebracht werden, bei der die thermische Spaltung stattfindet. Als Wandmaterial, das während der thermischen Spaltung mit den Gasen in Berührung kommt, eignet sich beispielsweise Nickel, hochnickelhaltige Stähle, Graphit und insbesondere Platin oder ähnliche Edelmetalle.

Nach der thermischen Spaltung wird das Gasgemisch zweckmäßig durch Einsprühen von Wasser schnell abgekühlt, nochmals mit Wasser gewaschen, dann mit wäßrigem Alkali, beispielsweise Natronlauge, behandelt, anschließend, beispielsweise mit konzentrierter Schwefelsäure, getrocknet und fraktioniert destilliert. Als hauptsächliches Reaktionsprodukt wird Hexafluorpropen gewonnen, das sich mit guter Selektivität gebildet hat. Die nicht-umgesetzten Anteile an Tetrafluorethen sowie gegebenenfalls Chlortetrafluorethan, Chlorhexafluorpropan beziehungsweise Perfluorcyclobutan werden in den Prozeß zurückgeführt. Unerwünschte Nebenprodukte werden nach bekannten Methoden entsorgt.

Wie oben bereits erwähnt, ermöglicht es das erfindungsgemäße Verfahren, Chlortetrafluorethan und/oder Chlorhexafluorpropan, insbesondere die bei der Herstellung von Tetrafluorethen hauptsächlich als Nebenprodukte anfallenden Verbindungen 1-Chlor-1,1,2,2-tetrafluorethan und 1-Chlor-1,1,2,2,3,3-hexafluorpropan, bei guter Selektivität mit wesentlich verbesserter Raum-Zeit-Ausbeute ohne Erhöhung der Spalttemperatur zu Hexafluorpropen umzusetzen. Das neue Verfahren ermöglicht ferner, Chlortetrafluorethan und Perfluorcyclobutan, insbesondere die bei der technischen Herstellung von Tetrafluorethen als Nebenprodukt anfallenden azeotropen Gemische aus Chlortetrafluorethan, bei guter Selektivität mit wesentlich verbesserter Raum-Zeit-Ausbeute ohne Erhöhung der Spalttemperatur zu Hexafluorpropen umzusetzen. Das neue Verfahren benötigt nur einen geringen apparativen Mehraufwand, verglichen mit der bekannten thermischen Spaltung von Chlortetrafluorethan oder Perfluorcyclobutan ohne Tetrafluorethenzusatz.

Nachfolgende Beispiele sollen die Erfindung näher erläutern:

Vergleichsversuche A bis E sowie Beispiele 1 bis 6

Die zur thermischen Spaltung vorgesehenen Stoffe Chlortetrafluorethan, Chlorhexafluorpropan, Gemische von Chlortetrafluorethan und Perfluorcyclobutan sowie Chlordifluormethan werden je einer Stahlflasche flüssig entnommen, in je einem mit Niederdruck-Wasserdampf beheizten Verdampfer verdampft und über je ein beheiztes Rotameter in eine beheizte Mischkammer geleitet. Die genauen Stoffmengen werden durch

4

Wägung der Stahlflaschen ermittelt. Tetrafluorethen wird der Mischkammer gasförmig über einen Durchflußmesser zugefahren. Aus der Mischkammer werden Proben entnommen und gaschromatographisch untersucht. Über eine mit Begleitheizung versehene Leitung wird der Mischkammer Wasserdampf zugeleitet, der in einem weiteren Verdampfer erzeugt wird. Die Menge des Wasserdampfes kann mit Hilfe einer pulsationsfreien Dosierpumpe (Typ MDP-600 von Firma Labomatic/Sinsheim) genau eingestellt werden.

Von der Mischkammer gelangt das wasserdampfhaltige Gasgemisch in den Reaktor, der aus einem U-förmig gebogenen Platinrohr von 4 mm Innendurchmesser und 0,25 mm Wandstärke besteht. Das Platinrohr ist in einem Röhrenofen (Typ ROK/F-4/140 von Firma Heraeus/Hanau) angebracht, der eine regelbare Heizleistung von maximal 3,75 kW hat und das Platinrohr auf einer Länge von 2 m beheizt. Das Innenvolumen des beheizten Rohrteiles beträgt 25,13 cm$^3$. Der Innendruck wird am Anfang des Platinrohres, die Temperatur kurz vor dem Ende des beheizten Rohrteiles gemessen.

Nach Durchlaufen des Reaktors wird das der thermischen Spaltung unterworfene Gasgemisch in eine Quenchkolonne (Diabon DN 50 mm, H = 1,05 m von Firma Sigri/Meitingen) geleitet und dort mit einer 15 Gew.-% HCl enthaltenden, wäßrigen Salzsäure von 20 °C abgeschreckt. Eine Kreiselpumpe fördert diese Salzsäure mit einer regelbaren Fördermenge von maximal 100 dm$^3$/h durch einen Wärmetauscher, der den Wärmeinhalt des Spaltgasgemisches abführt, in einen Abscheider, in dem Gasgemisch und Flüssigkeit getrennt werden. Der HCl-Gehalt dieser Flüssigkeit wird durch Ausschleusen eines Teils der Salzsäure und Ergänzen des ausgeschleusten Flüssigkeitsvolumens durch Wasser konstant gehalten und die nun wieder 15 Gew.-% HCl enthaltende Salzsäure der Quenchkolonne erneut zugeführt. Das Spaltgasgemisch wird nach Verlassen des Abscheiders mit Wasser gewaschen, anschließend zur Trocknung durch Schwefelsäure geleitet und dann in einen Gasbehälter gegeben, aus dem Proben zur gaschromatographischen Analyse genommen werden.

Die gaschromatographischen Analysen werden auf einem Hewlett Packard 5890 A mit WLD durchgeführt, an dem ein Shimadzu-Integrator C-R3A angeschlossen ist. Der Gaschromatograph ist mit einer Stahlsäule (10 m, 1/8 Zoll) ausgerüstet, die mit Porasil C (80 bis 100 mesh, von Firma Amchro, Sulzbach/Taunus, Bundesrepublik Deutschland) gepackt ist. Folgendes Temperaturprogramm wird angewendet:

| | |
|---|---|
| Anfangstemperatur: | 30 °C, 7 min isotherm |
| Aufheizrate: | 1 grd/min |
| Endtemperatur: | 100 °C |

Als Trägergas dient Helium (20 ml/min), das Dosiervolumen beträgt 50 bis 100 μl. Die gemessenen Flächenprozente werden in Gewichtsprozente umgerechnet und daraus die eingesetzten sowie die nach der thermischen Spaltung erhaltenen Gasmengen in g/h ermittelt.

Die Versuchsergebnisse sind zur besseren Übersicht in nachfolgender Tabelle aufgeführt. Darin bedeuten:

```
F124      = Gemisch aus 86 Gew.-%
            1-Chlor-1,1,2,2-tetrafluorethan; 14 Gew.-%
            2-Chlor-1,1,1,2-tetrafluorethan
```

F226 = 1-Chlor-1,1,2,2,3,3-hexafluorpropan

$C_4F_8$-c = Perfluorcyclobutan

TFE = Tetrafluorethen

F22 = Chlordifluormethan

Rest I = im wesentlichen höher als Hexafluorpropen siedende, fluorierte, teilweise chlor- und wasserstoffhaltige, geradkettige, cyclische, gesättigte und ungesättigte Kohlenwasserstoffe

Rest II = wie Rest I, jedoch zusätzlich Chlorwasserstoff und geringe Mengen Fluorwasserstoff sowie weitere fluorierte, teilweise chlor- und wasserstoffhaltige, geradkettige, cyclische, gesättigte und ungesättigte Kohlenwasserstoffe, die sich bei der Reaktion als Nebenprodukte bilden

TABELLE

| Beispiel Vergleichs-versuch | Eingesetztes Gas (g/h) | | | | | | | | mol TFE/ mol F124 + $C_4F_8$-c | mol TFE/ mol F124 + F226 | mol $H_2O$/ mol TFE + F124 + $C_4F_8$-c | mol $H_2O$/ mol TFE + F124 + F226 |
|---|---|---|---|---|---|---|---|---|---|---|---|---|
| | F124 | $C_4F_8$-c | F226 | TFE | F22 | $H_2O$ | Rest I | Gesamt | | | | |
| A | 439,0 | - | 1,5 | 0 | 0,3 | 47 | 29,2 | 517 | - | 0 | - | 0,81 |
| 1 | 428,7 | - | 1,6 | 416,4 | 0 | 47 | 38,3 | 932 | - | 1,32 | - | 0,36 |
| B [7] | 249,5 | - | 0,4 | 0 | 266,2 | 27 | 20,9 | 564 | 0 | 0 | 0,82 | 0,82 |
| C | 0 | - | 355,4 | 0 | 0 | 60 | 264,6 | 680 | - | 0 | - | 1,75 |
| 2 | 0 | - | 371,6 | 491,9 | 0,5 | 60 | 246,0 | 1 170 | - | 2,47 | - | 0,48 |
| D | 237,3 | - | 152,1 | 0 | 0,2 | 55 | 160,4 | 605 | - | 0 | - | 1,19 |
| 3 | 221,7 | - | 156,1 | 538,8 | 0,8 | 55 | 172,6 | 1 145 | - | 2,19 | - | 0,39 |
| 4 | 147,7 | - | 91,2 | 352,4 | 0 | 109 | 78,7 | 779 | - | 2,24 | - | 1,19 |
| E | 128,4 | 331,7 | - | 0 | - | 58 | 119,9 | 638 | 0 | - | 1,24 | - |
| 5 | 92,5 | 222,5 | - | 368,2 | - | 41 | 91,8 | 816 | 2,06 | - | ,0,42 | - |
| 6 | 136,2 | 333,4 | - | 788,6 | - | 58 | 141,8 | 1 458 | 2,96 | - | 0,31 | - |

EP 0 337 127 B1

TABELLE (Fortsetzung)

| Beispiel Vergleichsversuch | Reaktor- temperatur °C 1) | Reaktor- druck kPa 2) | Partial- druck kPa 3) | mittlere Verweil- zeit s 4) | Erzeugtes Gas (g/h) | | | | | | | Selek- tivität HFP % 6) | Raum- Zeit Ausbeute HFP 5) |
|---|---|---|---|---|---|---|---|---|---|---|---|---|---|
| | | | | | HFP | F124 | C₄F₈-c | F226 | TFE | F22 | Rest II + H₂O | | |
| A | 850 | 109,7 | 59,0 | 0,18 | 66,8 | 114,2 | - | 2,6 | 112,2 | 21,6 | 199,6 | 27,8 | 2,64 |
| 1 | 860 | 112,5 | 81,1 | 0,10 | 169,6 | 152,0 | - | 3,2 | 284,8 | 22,5 | 299,9 | 50,5 | 6,72 |
| B 7) | 850 | 110,4 | 31,1 | 0,16 | 90,8 | 51,6 | 10,1 | 4,1 | 122,2 | 37,2 | 258,1 | 32,7 | 3,61 |
| C | 840 | 110,5 | 32,3 | 0,16 | 64,0 | 0 | - | 111,0 | 95,3 | 11,9 | 397,8 | 31,7 | 2,48 |
| 2 | 860 | 116,1 | 69,9 | 0,09 | 315,7 | 0 | - | 74,8 | 203,4 | 11,6 | 564,5 | 59,9 | 12,56 |
| D | 850 | 109,0 | 43,6 | 0,17 | 66,7 | 61,4 | - | 44,1 | 110,4 | 18,7 | 303,7 | 30,8 | 2,65 |
| 3 | 850 | 115,9 | 77,0 | 0,09 | 277,0 | 69,9 | - | 45,1 | 305,4 | 20,4 | 427,2 | 65,6 | 10,98 |
| 4 | 860 | 113,1 | 49,8 | 0,09 | 173,7 | 45,3 | - | 27,4 | 154,2 | 9,7 | 368,7 | 53,3 | 7,21 |
| E | 840 | 109,4 | 44,3 | 0,17 | 99,8 | 56,6 | 54,2 | - | 180,3 | 8,5 | 238,6 | 29,9 | 3,93 |
| 5 | 850 | 112,6 | 74,8 | 0,13 | 241,3 | 43,8 | 50,0 | - | 258,0 | 5,5 | 217,4 | 75,6 | 9,58 |
| 6 | 850 | 118,4 | 85,8 | 0,07 | 251,4 | 100,3 | 207,5 | - | 636,3 | 4,4 | 258,1 | 82,3 | 9,97 |

1) am Reaktorausgang gemessen
2) am Reaktoreingang gemessen
3) Partialdruck des Gasgemisches aus F124 + F226 + TFE
4) bezogen auf Volumen der eingesetzten Molzahl bei Reaktortemperatur und einem Druck von 100 kPa
5) in g HFP je cm³ Reaktionsraum und Stunde
6) berechnet auf Basis der Kohlenstoffatom-Bilanz
7) nach DE-PS 1 236 497

## Patentansprüche

1. Verfahren zur Herstellung von Hexafluorpropen, dadurch gekennzeichnet, daß man Chlortetrafluorethan und/oder Chlorhexafluorpropan oder ein Gemisch von Chlortetrafluorethan und Perfluorcyclobutan bei einer Temperatur von 600 bis 1 000 °C und einem Druck von 1 bis 1 000 kPa in Gegenwart von 0,05 bis 20 mol Tetrafluorethen auf 1 mol eingesetztes Chlortetrafluorethan und/oder Chlorhexafluorpropan oder Gemisch aus Chlortetrafluorethan und Perfluorcyclobutan thermisch spaltet.

8

**2.** Verfahren nach Anspruch 1, dadurch gekennzeichnet, daß die thermische Spaltung bei 700 bis 900 °C erfolgt.

**3.** Verfahren nach Anspruch 1 oder 2, dadurch gekennzeichnet, daß das verwendete Chlortetrafluorethan höchstens 20 Gew.-%, bezogen auf dessen Gesamtmenge, 2-Chlor-1,1,1,2-tetrafluorethan enthält.

**4.** Verfahren nach einem oder mehreren der Ansprüche 1 bis 3, dadurch gekennzeichnet, daß die thermische Spaltung in Gegenwart von 0,5 bis 5 mol Tetrafluorethen auf 1 mol eingesetztes Chlortetrafluorethan und/oder Chlorhexafluorpropan erfolgt.

**5.** Verfahren nach einem oder mehreren der Ansprüche 1 bis 3, dadurch gekennzeichnet, daß die thermische Spaltung in Gegenwart von 0,5 bis 5 mol Tetrafluorethen auf 1 mol eingesetztes Gemisch aus Chlortetrafluorethan und Perfluorcyclobutan erfolgt.

**6.** Verfahren nach einem oder mehreren der Ansprüche 1 bis 4, dadurch gekennzeichnet, daß die Verweilzeit des Gasgemisches aus Tetrafluorethen, Chlortetrafluorethan und/oder Chlorhexafluorpropan bei 600 bis 1 000 °C 0,01 bis 20 Sekunden beträgt.

**7.** Verfahren nach einem oder mehreren der Ansprüche 1 bis 3 und 5, dadurch gekennzeichnet, daß die Verweilzeit des Gasgemisches aus Tetrafluorethen, Chlortetrafluorethan und Perfluorcyclobutan bei 600 bis 1 000 °C 0,01 bis 20 Sekunden beträgt.

**Claims**

**1.** Process for the preparation of hexafluoropropene, characterized by thermally cleaving of chlorotetrafluoroethane and/or chlorohexafluoropropane or a mixture of chlorotetrafluoroethane and perfluorocyclobutane at a temperature from 600 to 1,000°C and a pressure of 1 to 1,000 kPa in the presence of 0.05 to 20 mole of tetrafluoroethane and/or chlorohexafluoropropane or a mixture of chlorotetrafluoroethane and perfluorocyclobutane.

**2.** Process as claimed in claim 1, wherein the thermal cleavage is carried out at 700 to 900°C.

**3.** Process as claimed in claim 1 or 2, wherein the chlorotetrafluoroethane used contains not more than 20% by weight, relative to the total amount thereof, of 2-chloro-1,1,1,2-tetrafluoroethane.

**4.** The process as claimed in one or more of claims 1 to 3, wherein the thermal cleavage is carried out in the presence of 0.5 to 5 moles of tetrafluoroethylene per mole of chlorotetrafluoroethane and/or chlorohexafluoropropane employed.

**5.** The process as claimed in one or more of claims 1 to 3, wherein the thermal cleavage is carried out in the presence of 0.5 to 5 moles of tetrafluoroethylene per mole of mixture of chlorotetrafluoroethane and perfluorocyclobutane employed.

**6.** The process as claimed in one or more of claims 1 to 4, wherein the dwell time of the gas mixture of tetrafluoroethylene, chlorotetrafluoroethane and/or chlorohexafluoropropane at 600 to 1,000°C is 0.01 to 20 seconds.

**7.** The process as claimed in one or more of claims 1 to 3 and 5, wherein the dwell time of the gas mixture of tetrafluoroethylene, chlorotetrafluoroethane and perfluorocyclobutane at 600 to 1,000°C is 0.01 to 20 seconds.

**Revendications**

**1.** Procédé de préparation d'hexafluoropropène, caractérisé en ce que l'on soumet à une dissociation sous l'action de la chaleur du chlorotétrafluoroéthane et/ou du chlorohexafluoropropane ou un mélange de chlorotétrafluoréthane et de perfluorocyclobutane à une température de 600 à 1000 °C sous une pression de 1 à 1000 kPa en présence de 0,05 à 20 mol de tétrafluoroéthène par mol du chlorotétrafluoroéthane et/ou du chlorohexafluoropropane ou du mélange de chlorotétrafluoréthane et de perfluorocy-

clobutane.

2. Procédé selon la revendication 1, caractérisé en ce que l'on opère à une température de 700 à 900 °C.

3. Procédé selon la revendication 1 ou 2, caractérisé en ce que le chlorotétrafluoréthane contient au maximum 20 %, de sa masse totale, de 2-chloro-1,1,1,2-tétrafluoréthane.

4. Procédé selon une ou plusieurs des revendications 1 à 3, caractérisé en ce que l'on effectue la dissociation en présence de 0,5 à 5 mol de tétrafluoréthène par mol du chlorotétrafluoréthane et/ou du chlorohexafluoropropane.

5. Procédé selon une ou plusieurs des revendications 1 à 3, caractérisé en ce que l'on effectue la dissociation en présence de 0,5 à 5 mol de tétrafluoréthène par mol du mélange de chlorotétrafluoréthane et de perfluorocyclobutane.

6. Procédé selon une ou plusieurs des revendications 1 à 4, caractérisé en ce que le temps de séjour à la température de 600 à 1000 °C du mélange gazeux de tétrafluoréthène, de chlorotétrafluoréthane et/ou de chlorohexafluoropropane est de 0,01 à 20 secondes.

7. Procédé selon une ou plusieurs des revendications 1 à 3 et 5, caractérisé en ce que le temps de séjour à la température de 600 à 1000 °C du mélange gazeux de tétrafluoréthène, de chlorotétrafluoréthane et de perfluorocyclobutane est de 0,01 à 20 secondes.